# EUROPEAN PATENT APPLICATION

(11) **EP 1 754 514 A1**
(43) Date of publication of application: **21.02.2007**
(21) Application number: 05004620.0
(22) Date of filing: 03.03.2005
(51) Int. Cl.: A61Q 5/10

(54) **Agent and method for colouring keratin fibres**

(71) Applicant: Wella Aktiengesellschaft, 64274 Darmstadt (DE)
(72) Inventor: Pasquier, Cécile, 1723 Marly (CH); Duc-Reichlin, Nadia, 1470 Lully (CH); Braun, Hans-Jürgen, 3182 Ueberstorf (CH)

(57) **Abstract**

The present invention provides an agent for colouring keratin fibres (A) which comprises at least one heterocyclic hydrazone derivative of the formula (I) or its physiologically compatible salt 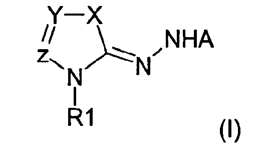 and at least one ortho-quinone of the formula (II) or para-quinone of the formula (III); 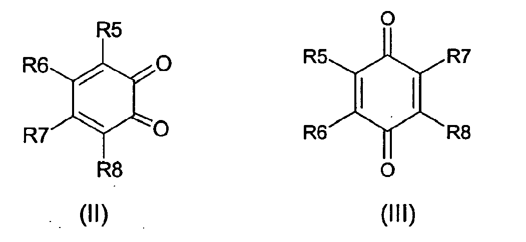 and a method of colouring hair using this agent.

## Description

The present application provides an agent for colouring keratin fibres, such as, for example, silk, wool or hair and in particular human hair, which comprises a combination of (i) at least one heterocyclic hydrazone derivative and (ii) at least one quinoid compound, and a method of colouring keratin fibres using this agent.

Hair colourants are primarily divided into the field of oxidation colourants and of tints depending on the starting hair colour to be coloured and the desired end result. Oxidation hair colours are exceptionally suitable for covering relatively large amounts of grey, with the oxidation colourants used for a grey content of up to 50% generally being referred to as oxidative tints, while the oxidation colourants used for a grey content of more than 50% or for "lightening" are generally referred to as so-called oxidative colours. Direct dyes are primarily present in nonoxidative colourants (so-called tinting agents). Some direct dyes, such as, for example, nitro dyes, can, on account of their small size, penetrate into the hair and colour it directly - at least in the outer regions. Such tints are very gentle on the hair and generally withstand 6 to 8 hair washes. Direct dyes are likewise often used in oxidative colourants for producing certain shades or for intensifying the colour.

EP-A 0 848 942, DE-A 43 35 624 and EP-B 0 460 996 have already described colouring systems based on benzoquinones, and compounds containing amino groups and/or hydroxyl groups. It is also known from WO 03/042199 to use a combination of 2-benzothiazolinone hydrazones and benzoquinones for the colouring of keratin fibres. It is likewise known from DE-A 103 09 523 to use certain heterocyclic hydrazone derivatives as developer substances in oxidation hair colourants. However, some of the colouring systems known to date are unable to satisfy the requirements placed on colourants in every respect. There therefore continues to be a great need for colourants which permit both intense and also gentle colourations especially under mild conditions.

Surprisingly, it has now been found that the use of a combination of (i) at least one heterocyclic hydrazone derivative of the formula (I) and (ii) at least one quinoid compound permits intensive colourations in the yellow to blue colour range in a gentle manner under mild conditions.

The present invention therefore provides an agent for colouring fibres (A), such as, for example, wool, silk, cotton or hair and in particular human hair, which is prepared prior to application by mixing two components (A1) and (A2) and is characterized in that component (A1) comprises at least one heterocyclic hydrazone derivative of the formula (I) or its physiologically compatible salt, 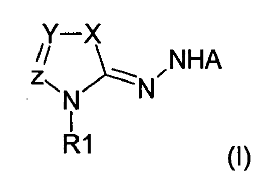 in which
**X** is oxygen, sulphur or N-R2,
**Y** is C-R3 or nitrogen and
**Z** is C-R4 or nitrogen,
with the proviso that the heterocyclic moiety in the formula (I) has at most three heteroatoms;
**A** is hydrogen, an acetyl group, a trifluoroacetyl group, a formyl group, a (C₁-C₆)-alkylsulphonyl group or an arylsulphonyl group;
**R1** and **R2** may be identical or different, and, independently of one another, are a saturated or unsaturated (C₁-C₁₂)-alkyl group, a (C₁-C₁₂)-alkyl group substituted by a halogen atom (F, Cl, Br, I), a hydroxy-(C₂-C₁₂)-alkyl group, an amino-(C₂-C₁₂)-alkyl group, a sulphonic acid-(C₁-C₁₂)-alkyl group, an isocyclic or heterocyclic group, a formyl group, a C(O)-alkyl group, a C(O)-phenyl group, a C(O)NH-alkyl group, a C(O)NH-phenyl group, a substituted or unsubstituted phenyl group or a substituted or unsubstituted benzyl group;
**R3** and **R4** may be identical or different and, independently of one another, are hydrogen, a halogen atom (F, Cl, Br, I), a saturated or unsaturated (C₁-C₁₂)-alkyl group, a (C₁-C₁₂)-alkyl group substituted by a halogen atom (F, Cl, Br, I), a hydroxyl group, a hydroxy-(C₁-C₁₂)-alkyl group, a (C₁-C₁₂)-alkoxy group, a (C₁-C₆)-alkoxy-(C₁-C₆)-aikyl group, a cyano group, a nitro group, an amino group, a (C₁-C₁₂)-alkylamino group, a (C₁-C₁₂)-dialkylamino group, a carboxylic acid, a C(O)O-(C₁-C₁₂)-alkyl group, a substituted or unsubstituted C(O)O-phenyl group, a substituted or unsubstituted phenyl group, a naphthyl group, a nonaromatic isocyclic group or a substituted or unsubstituted heteroaryl group;
and if **Y** and **Z** are C-R3 and C-R4, **R3** and **R4,** together with the residual molecule, can form a heterocyclic, saturated or unsaturated, substituted or unsubstituted ring system;
and the component (A2) comprises at least one ortho-quinone of the formula (II) or para-quinone of the formula (III), 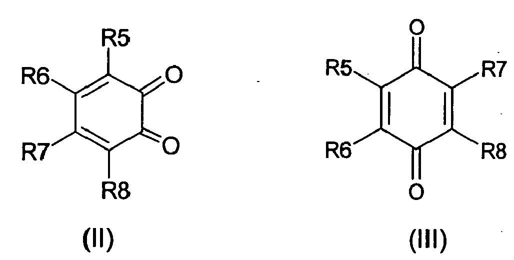
where **R5, R6, R7** and **R8,** independently of one another, are hydrogen, a halogen atom (F, Cl, Br, I), a (C₁-C₁₂)-alkyl group substituted by a halogen atom (F, Cl, Br, I), a hydroxyl group, a hydroxy-(C₁-C₁₂)-alkyl group, a polyhydroxy-(C₂-C₁₂)-alkyl group, a (C₁-C₁₂)-alkoxy group, a mono-(C₁-C₆)-alkoxy-(C₁-C₆)-alkyl group, a poly-(C₁-C₆)-alkoxy-(C₂-C₆)-alkyl group, a (C₁-C₂)-alkylenedioxy group, a cyano group, a nitro group, a sulphonic acid group, a carboxylic acid, a C(O)O-(C₁-C₁₂)-alkyl group, a substituted or unsubstituted C(O)O-phenyl group, a substituted or unsubstituted phenyl group, a naphthyl group, a nonaromatic isocyclic group, or an aromatic or nonaromatic heterocyclic group, or an amino group **-NR9R10,** where the radicals **R9** and **R10** may be identical or different and, independently of one another, are hydrogen, a (C₁-C₁₂)-alkyl group, a carbocyclic or heterocyclic substituted aromatic compound or a carbocyclic or heterocyclic unsubstituted aromatic compound, or **R9** and **R10,** together with the nitrogen atom, form a saturated or unsaturated, substituted or unsubstituted heterocyclic (C₃-C₆)-radical (for example an imidazolidino, piperidino, pyrrolidino, pyrazolidino, piperazino or morpholino group);
or in the case of o-quinones of the formula (II) **R5** and **R6** and/or **R6** and **R7** and/or **R7** and **R8,** or in the case of p-quinones of the formula (III) **R5** and **R6,** and/or **R7** and **R8,** in each case together with the remaining molecule, can form a heterocyclic or carbocyclic, substituted or unsubstituted ring.

Particularly preferred are hydrazone derivatives of the formula (I) or physiologically compatible salts thereof in which **X** is sulphur, **Y** is C-R3, **Z** is C-R4 and **A** is a hydrogen atom.

Examples of the compounds of the formula (I) which may be mentioned are the following compounds:3-methyl-2(3H)-thiazolone hydrazone, 3,4-dimethyl-2(3H)-thiazolone hydrazone, 4-tert-butyl-3-methyl-2(3H)-thiazolone hydrazone, 3-methyl-4-phenyl-2(3H)-thiazolone hydrazone, 3-methyl-4-(4-tolyl)-2(3H)-thiazolone hydrazone, 4-(4-methoxy)phenyl-3-methyl-2(3H)-thiazolone hydrazone, 4-(4-ethoxy)phenyl-3-methyl-2(3H)-thiazolone hydrazone, 4-(4-bromophenyl)-3-methyl-2(3H)-thiazolone hydrazone, 4-(3-bromophenyl)-3-methyl-2(3H)-thiazolone hydrazone, 4-(4-chlorophenyl)-3-methyl-2(3H)-thiazolone hydrazone, 4-(3-chlorophenyl)-3-methyl-2(3H)-thiazolone hydrazone, 3-methyl-4-(4-nitrophenyl)-2(3H)-thiazolone hydrazone, 3-methyl-4-(3-nitrophenyl)-2(3H)-thiazolone hydrazone, 4-([1,1'-biphenyl]-4-yl)-3-methyl-2(3H)-thiazolone hydrazone, 3-methyl-4-(2-naphthalenyl)-2(3H)-thiazolone hydrazone, ethyl 2-hydrazono-2,3-dihydro-3-methyl-4-thiazolecarboxylate, 3,4,5-trimethyl-2(3H)-thiazolone hydrazone, 3,4-dimethyl-5-phenyl-2(3H)-thiazolone hydrazone, 3,5-dimethyl-4-phenyl-2(3H)-thiazolone hydrazone, 3-methyl-4,5-diphenyl-2(3H)-thiazolone hydrazone, 5-ethyl-3-methyl-4-phenyl-2(3H)-thiazolone hydrazone, 4-(4-bromophenyl)-3-methyl-5-phenyl-2(3H)-thiazolone hydrazone, 3-methyl-5-phenyl-4-(4-tolyl)-2(3H)-thiazolone hydrazone, 5-(4-chlorophenyl)-4-phenyl-3-methyl-2(3H)-thiazolone hydrazone, 5-(4-chlorophenyl)-4-(4-methoxyphenyl)-3-methyl-2(3H)-thiazolone hydrazone, ethyl 2-hydrazono-2,3-dihydro-3,4-dimethyl-4-thiazolecarboxylate, 4-amino-2-hydrazono-2,3-dihydro-3-methyl-5-thiazolecarbonitrile, 3-ethyl-4,5-dimethyl-2(3H)-thiazolone hydrazone, ethyl 2-hydrazono-2,3-dihydro-3-ethyl-4-methyl-thiazolecarboxylate, 5-methyl-3-(1-methylethyl)-4-phenyl-2(3H)-thiazolone hydrazone, 4,5-dimethyl-3-(1-methylethyl)-2(3H)-thiazolone hydrazone, 3-(1-methylethyl)-4,5-diphenyl-2(3H)-thiazolone hydrazone, 4,5-dimethyl-3-propyl-2(3H)-thiazolone hydrazone, 4,5-diphenyl-3-propyl-2(3H)-thiazolone hydrazone, 3-butyl-4,5-dimethyl-2(3H)-thiazolone hydrazone, 3-butyl-4,5-diphenyl-2(3H)-thiazolone hydrazone, 4,5-dimethyl-3-(2-methylpropyl)-2(3H)-thiazolone hydrazone, 3-(2-methylpropyl)-4,5-diphenyl-2(3H)-thiazolone hydrazone, 3-hydroxyethyl-2(3H)-thiazolone hydrazone, 3-hydroxyethyl-4-methyl-2(3H)-thiazolone hydrazone, 3-hydroxyethyl-4,5-dimethyl-2(3H)-thiazolone hydrazone, 3-aminoethyl-2(3H)-thiazolone hydrazone,3-aminoethyl-4-methyl-2(3H)-thiazolone hydrazone, 3-aminoethyl-4,5-dimethyl-2(3H)-thiazolone hydrazone, 3,4-diphenyl-2(3H)-thiazolone hydrazone, 4-methyl-3-phenyl-2(3H)-thiazolone hydrazone, 4,5-dimethyl-3-phenyl-2(3H)-thiazolone hydrazone, 4-p-biphenylyl-3-phenyl-2(3H)-thiazolone hydrazone, 4-(4-methoxy)phenyl-3-phenyl-2(3H)-thiazolone hydrazone, 4-tert-butyl-3-phenyl-2(3H)-thiazolone hydrazone, 5-methyl-3,4-diphenyl-2(3H)-thiazolone hydrazone, 3,4,5-triphenyl-2(3H)-thiazolone hydrazone, 4,5-dimethyl-3-(phenylmethyl)-2(3H)-thiazolone hydrazone, 3-(2-propenyl)-2(3H)-thiazolone hydrazone, 4-methyl-3-(2-propenyl)-2(3H)-thiazolone hydrazone, 4-tert-butyl-3-(2-propenyl)-2(3H)-thiazolone hydrazone, 4-phenyl-3-(2-propenyl)-2(3H)-thiazolone hydrazone, 4,5-dimethyl-3-(2-propenyl)-2(3H)-thiazolone hydrazone, 4,5-diphenyl-3-(2-propenyl)-2(3H)-thiazolone hydrazone, ethyl 2-hydrazono-2,3-dihydro-3-[(phenylamino)carbonyl]-4-methylthiazolecarboxylate, 3-methyl-2(3H)-oxazolone hydrazone, 3-phenyl-2(3H)-oxazolone hydrazone, 1,3-dimethyl-1,3-dihydro-2H-imidazol-2-one hydrazone, 1,3-diethyl-1,3-dihydro-2H-imidazol-2-one hydrazone, 1,3-dihydroxyethyl-1,3-dihydro-2H-imidazol-2-one hydrazone, 1,3-diaminoethyl-1,3-dihydro-2H-imidazol-2-one hydrazone, 1,3-dimethyl-4-methoxy-1,3-dihydro-2H-imidazol-2-one hydrazone, 1,3,4-trimethyl-1,3-dihydro-2H-imidazol-2-one hydrazone, 1,3-dimethyl-1,3-dihydro-2H-imidazol-2-one hydrazone, 4-carboxy-1,3-dimethyl-1,3-dihydro-2H-imidazol-2-one hydrazone, 4-amino-1,3-dimethyl-1,3-dihydro-2H-imidazol-2-one hydrazone, 1,3-dimethyl-4-dimethylamino-1,3-dihydro-2H-imidazol-2-one hydrazone, 1,4-dimethyl-Δ2-1,2,4-triazolin-5-one hydrazone, 1,4-dihydroxyethyl-Δ2-1,2,4-triazolin-5-one hydrazone, 1,4-diaminoethyl-Δ2-1,2,4-triazolin-5-one hydrazone, 1,3,4-trimethyl-Δ2-1,2,4-triazolin-5-one hydrazone, 1,4-dimethyl-3-phenyl-Δ2-1,2,4-triazolin-5-one hydrazone, 1,4-dimethyl-3-methoxy-Δ2,-1,2,4-triazolin-5-one hydrazone, 1,4-dimethyl-3-dimethylamino-Δ2-1,2,4-triazolin-5-one hydrazone, 4-carboxy-1,4-dimethyl-Δ2-1,2,4-triazolin-5-one hydrazone, 4-amino-1,4-dimethyl-Δ2-1,2,4-triazolin-5-one hydrazone, 4-butyl-1-methyl-3-phenyl-Δ2-1,3,4-triazolin-5-one hydrazone, 1,4-dimethyl-Δ2-1,2,4-triazolin-5-one hydrazone, 1,4-dihydroxyethyl-Δ2-1,2,4-triazolin-5-one hydrazone, 1,4-aminoethyl-Δ2-1,2,4-triazolin-5-one hydrazone, 1,3,4-trimethyl-Δ2-1,2,4-triazolin-5-one hydrazone, 1,4-dimethyl-3-phenyl-Δ2-1,2,4-triazolin-5-one hydrazone, 4-methyl-3-phenyl-Δ2-1,2,4-triazolin-5-one hydrazone, 4-methyl-Δ2-1,3,4-thiadiazolin-5-one hydrazone, 4-hydroxyethyl-Δ2-1,3,4-thiadiazolin-5-one hydrazone, 4-aminoethyl-Δ2-1,3,4-thiadiazolin-5-one hydrazone, 4-methyl-2-phenyl-Δ2-1,3,4-thiadiazolin-5-one hydrazone, 2-methoxy-4-methyl-Δ2-1,3,4-thiadiazolin-5-one hydrazone, 2-anilino-4-methyl-Δ2-1,3,4-thiadiazolin-5-one hydrazone, 2-amino-4-methyl-Δ2-1,3,4-thiadiazolin-5-one hydrazone, 2-dimethylamino-4-methyl-Δ2-1,3,4-thiadiazolin-5-one hydrazone, 4-methyl-2-(methylthio)-Δ2-1,3,4-thiadiazolin-5-one hydrazone, 4-(5-hydrazono-4,5-dihydro-4-methyl-1,3,4-thiadiazol-2-yl)-benzenesulphonyl fluoride, 4-methyl-Δ2-1,2,4-thiadiazolin-5-one hydrazone, 4-hydroxyethyl-Δ2-1,2,4-thiadiazolin-5-one hydrazone, 4-aminoethyl-Δ2-1,2,4-thiadiazolin-5-one hydrazone, 4-methyl-3-phenyl-Δ2-1,2,4-thiadiazolin-5-one hydrazone, 3-methoxy-4-methyl-Δ2-1,2,4-thiadiazolin-5-one hydrazone, 3-amino-4-methyl-Δ2-1,2,4-thiadiazolin-5-one hydrazone, 3-dimethylamino-4-methyl-Δ2-1,2,4-thiadiazolin-5-one hydrazone and 3-carboxy-4-methyl-Δ2-1,2,4-thiadiazolin-5-one hydrazone.

Among the compounds of the formula (I), particular preference is given to the following thiazolone hydrazone derivatives:
3-methyl-2(3H)-thiazolone hydrazone, 3,4-dimethyl-2(3H)-thiazolone hydrazone, 4-tert-butyl-3-methyl-2(3H)-thiazolone hydrazone, 3-methyl-4-phenyl-2(3H)-thiazolone hydrazone, 3-methyl-4-(4-tolyl)-2(3H)-thiazolone hydrazone, 4-(4-methoxy)phenyl-3-methyl-2(3H)-thiazolone hydrazone, 4-(4-ethoxy)phenyl-3-methyl-2(3H)-thiazolone hydrazone, 4-(4-bromophenyl)-3-methyl-2(3H)-thiazolone hydrazone, 4-(3-bromophenyl)-3-methyl-2(3H)-thiazolone hydrazone, 4-(4-chlorophenyl)-3-methyl-2(3H)-thiazolone hydrazone, 4-(3-chlorophenyl)-3-methyl-2(3H)-thiazolone hydrazone, 3-methyl-4-(4-nitrophenyl)-2(3H)-thiazolone hydrazone, 3-methyl-4-(3-nitrophenyl)-2(3H)-thiazolone hydrazone, 4-([1,1'-biphenyl]-4-yl)-3-methyl-2(3H)-thiazolone hydrazone, ethyl 2-hydrazono-2,3-dihydro-3-methyl-4-thiazolecarboxylate, 3,4,5-trimethyl-2(3H)-thiazolone hydrazone, 3,4-dimethyl-5-phenyl-2(3H)-thiazolone hydrazone, 3,5-dimethyl-4-phenyl-2(3H)-thiazolone hydrazone, 3-methyl-4,5-diphenyl-2(3H)-thiazolone hydrazone, 5-ethyl-3-methyl-4-phenyl-2(3H)-thiazolone hydrazone, 4-(4-bromophenyl)-3-methyl-5-phenyl-2(3H)-thiazolone hydrazone, 3-methyl-5-phenyl-4-(4-tolyl)-2(3H)-thiazolone hydrazone, 5-(4-chlorophenyl)-4-phenyl-3-methyl-2(3H)-thiazolone hydrazone, 5-(4-chlorophenyl)-4-(4-methoxyphenyl)-3-methyl-2(3H)-thiazolone hydrazone, ethyl 2-hydrazono-2,3-dihydro-3,4-dimethyl-4-thiazolecarboxylate, 4-amino-2-hydrazono-2,3-dihydro-3-methyl-5-thiazolecarbonitrile, 3-ethyl-4,5-dimethyl-2(3H)-thiazolone hydrazone, ethyl 2-hydrazono-2,3-dihydro-3-ethyl-4-methyl-thiazolecarboxylate, 5-methyl-3-(1-methylethyl)-4-phenyl-2(3H)-thiazolone hydrazone, 4,5-dimethyl-3-(1-methylethyl)-2(3H)-thiazolone hydrazone, 3-(1-methylethyl)-4,5-diphenyl-2(3H)-thiazolone hydrazone, 4,5-dimethyl-3-propyl-2(3H)-thiazolone hydrazone, 4,5-diphenyl-3-propyl-2(3H)-thiazolone hydrazone, 3-butyl-4,5-dimethyl-2(3H)-thiazolone hydrazone, 3-butyl-4,5-diphenyl-2(3H)-thiazolone hydrazone, 4,5-dimethyl-3-(2-methylpropyl)-2(3H)-thiazolone hydrazone, 3-(2-methylpropyl)-4,5-diphenyl-2(3H)-thiazolone hydrazone, 3-hydroxyethyl-2(3H)-thiazolone hydrazone, 3-hydroxyethyl-4-methyl-2(3H)-thiazolone hydrazone, 3-hydroxyethyl-4,5-dimethyl-2(3H)-thiazolone hydrazone, 3-aminoethyl-2(3H)-thiazolone hydrazone, 3-aminoethyl-4-methyl-2(3H)-thiazolone hydrazone, 3-aminoethyl-4,5-dimethyl-2(3H)-thiazolone hydrazone, 3,4-diphenyl-2(3H)-thiazolone hydrazone, 4-methyl-3-phenyl-2(3H)-thiazolone hydrazone, 4,5-dimethyl-3-phenyl-2(3H)-thiazolone hydrazone, 4-p-biphenylyl-3-phenyl-2(3H)-thiazolone hydrazone, 4-(4-methoxy)phenyl-3-phenyl-2(3H)-thiazolone hydrazone, 4-tert-butyl-3-phenyl-2(3H)-thiazolone hydrazone, 5-methyl-3,4-diphenyl-2(3H)-thiazolone hydrazone, 3,4,5-triphenyl-2(3H)-thiazolone hydrazone, 4,5-dimethyl-3-(phenylmethyl)-2(3H)-thiazolone hydrazone, 3-(2-propenyl)-2(3H)-thiazolone hydrazone, 4-methyl-3-(2-propenyl)-2(3H)-thiazolone hydrazone, 4-tert-butyl-3-(2-propenyl)-2(3H)-thiazolone hydrazone, 4-phenyl-3-(2-propenyl)-2(3H)-thiazolone hydrazone, 4,5-dimethyl-3-(2-propenyl)-2(3H)-thiazolone hydrazone, 4,5-diphenyl-3-(2-propenyl)-2(3H)-thiazolone hydrazone and ethyl 2-hydrazono-2,3-dihydro-3-[(phenylamino)carbonyl]-4-methylthiazolecarboxylate.

The compounds of the formula (I) can be prepared by the synthesis processes known from the literature, for example the procedure in Research Disclosure October 1978, page 42 - 44, No. 17434 or analogously to the processes described in WO 02/074268 and DE-B 1 049 381, and the process described in Journal of Chemical Research, Synopses (1998), page 12-13.

Preferred ortho-quinones of the formula (II) are 4,5-dimethoxy-3,5-cyclohexadiene-1,2-dione, 5(5H),6(6H)-dioxo-1,3-benzodioxol, 2,3-dihydro-1,4-dimethyl-3-hydroxy-1 H-indole-5,6-dione, 2,3-dihydro-3-hydroxy-4-methoxy-1-methyl-1H-indole-5,6-dione, 3,5-di(1,1-dimethylethyl)-3,5-cyclohexadiene-1,2-dione, 2,3-dihydro-3-hydroxy-1-methy)-1 H-indole-5,6-dione, 3,4,5,6-tetrachloro-3,5-cyclohexadiene-1,2-dione, 4,5-dimethoxy-3,6-diphenyl-3,5-cyclohexadiene-1,2-dione, 4,5-di(phenylamino)-3,5-cyclohexadiene-1,2-dione, cacotheline, 3,4-dihydro-3,4-dioxo-1-naphthalenesulphonic acid ammonium salt, 4-methoxy-1,2-naphthalenedione, 1,2-naphthalenedione, 1,2-naphthalenedione-4-sulphonic acid sodium salt, 4-amino-1,2-naphthalenedione hydrate (2:1) and 9,10-phenanthrenequinone, particular preference being given to 4,5-dimethoxy-3,5-cyclohexadiene-1,2-dione, 5(5H),6(6H)-dioxo-1,3-benzodioxol, 2,3-dihydro-1,4-dimethyl-3-hydroxy-1H-indole-5,6-dione, 3,5-di(1,1-dimethylethyl)-3,5-cyclohexadiene-1,2-dione, 3,4,5,6-tetrachloro-3,5-cyclohexadiene-1,2-dione, cacotheline, 1,2-naphthalenedione, 1,2-naphthalenedione-4-sulphonic acid sodium salt, 4-amino-1,2-naphthalenedione hydrate (2:1) and 9,10-phenanthrenequinone.

Preferred para-quinones of the formula (III) are 1,4-benzoquinone, 2-methyl-1,4-benzoquinone, 2-tert-butyl-1,4-benzoquinone, 2-phenyl-1,4-benzoquinone, 2,5-dimethyl-1,4-benzoquinone, 2,6-dimethyl-1,4-benzoquinone, 2-methyl-5-(1-methylethyl)-1,4-benzoquinone, 2,5-di-tert-butyl-1,4-benzoquinone, 2,6-di-tert-butyl-1,4-benzoquinone, 2,5-diphenyl-1,4-benzoquinone, 2,3,5,6-tetramethyl-1,4-benzoquinone, 2-methoxy-1,4-benzoquinone, 2-methoxy-5-methyl-1,4-benzoquinone, 2,5-dimethoxy-1,4-benzoquinone, 2,6-dimethoxy-1,4-benzoquinone, 2,3-dimethoxy-5-methyl-1,4-benzoquinone, 2,6-dimethoxy-5-methyl-1,4-benzoquinone, 2,5-dihydroxy-1,4-benzoquinone, 2,5-dihydroxy-3-methyl-1,4-benzoquinone, 2,5-dihydroxy-3-methoxy-6-methyl-1,4-benzoquinone, 2,5-dihydroxy-3,6-diphenyl-1,4-benzoquinone, 3-hydroxy-2-methoxy-5-methyl-1,4-benzoquinone, 2-hydroxymethyl-6-methoxy-1,4-benzoquinone, 2,3,5,6-tetrahydroxy-1,4-benzoquinone, 2-bromo-1,4-benzoquinone, 2-chloro-1,4-benzoquinone, 2-fluoro-1,4-benzoquinone, 2-bromo-5-methyl-1,4-benzoquinone, 2,5-dibromo-1 ,4-benzoquinone, 2,5-dibromo-3,6-diphenyl-1,4-benzoquinone, 2,5-dibromo-3-methyl-6-(1-methylethyl)-1,4-benzoquinone, 2-chloro-6-methyl-1,4-benzoquinone, 2,5-dichloro-1,4-benzoquinone, 2,6-dichloro-1,4-benzoquinone, 2,5-dichloro-3,6-dimethyl-1,4-benzoquinone, 2,3-dichloro-5,6-dicyano-1,4-benzoquinone, 3,6-dioxocyclohexa-1,4-diene-1,2-dicarbonitrile, 2,5-dichloro-3,6-dioxocyclohexa-1,4-diene-1,4-dicarbonitrile, 2,5-dichloro-3,6-dihydroxy-1,4-benzoquinone, 2,5-dichloro-3,6-dimethylamino-1,4-benzoquinone, 2,3,5,6-tetrabromo-1,4-benzoquinone, 2,3,5,6-tetrachloro-1,4-benzoquinone, 2,3,5,6-tetrafluoro-1,4-benzoquinone, 2-amino-5-methyl-1,4-benzoquinone, 5-amino-2-chloro-1 ,4-benzoquinone, 2,5-di((2-hydroxyethyl)amino)-1,4-benzoquinone, 2,5-diamino-3,6-dichloro-1,4-benzoquinone, 1,4-naphthalenedione, 5,8-dihydroxy-1,4-naphthalenedione and 1 H-indole-4,7-dione, particular preference being given to 1,4-benzoquinone, 2-methyl-1,4-benzoquinone, 2,5-diphenyl-1,4-benzoquinone, 2,5-dihydroxy-1,4-benzoquinone, 2,3,5,6-tetrahydroxy-1,4-benzoquinone, 2,5-di((2-hydroxyethyl)amino)-1,4-benzoquinone, 1,4-naphthalenedione, 5,8-dihydroxy-1,4-naphthalenedione and 1 H-indole-4,7-dione.

The compounds of the formula (I) and the ortho- or para-quinones of the formula (II) or (III) are generally stored separate from one another and mixed together shortly prior to application. It is, however, also possible, if the compounds of the formula (I) and the ortho- or para-quinones of the formula (II) or (III) are in solid form, to package these together and to prepare the ready-to-use colourant (A) shortly prior to application by mixing the solid preparation (A') comprising the compounds of the formula (I) and the ortho- or para-quinones of the formula (II) or (III) with water or a liquid preparation (A") comprising the other constituents of the agent.

In addition, the colourant according to the invention can, in addition to the compounds of the formula (I) and the ortho-quinones of the formula (II) and/or para-quinones of the formula (III) in the component (A2) and (A') and the ready-to-use preparation (A), optionally additionally comprise further customary physiologically acceptable direct dyes from the group of cationic and anionic dyes, dispersion dyes, azo dyes, quinone dyes and triphenylmethane dyes.

These direct dyes can be used in component (A2) or (A') in a total amount of from about 0.02 to 20 per cent by weight, preferably 0.2 to 10 per cent by weight, the total amount of direct dyes in the ready-to-use colourant (A) being about 0.01 to 10 per cent by weight, preferably 0.1 to 5 per cent by weight.

The colourant according to the invention generally consists of a mixture of components (A1) and (A2), namely a colour carrier mass (A1) which comprises one or more compounds of the formula (I), and a further colour carrier mass (A2) which comprises one or more quinoid compounds of the formula (II)/(III).

The compounds of the formula (I) and the ortho-quinones of the formula (II) and/or para-quinones of the formula (III) are present in the particular colour carrier mass (component (A1) or component (A2) or component (A')) in each case in a total amount of from about 0.02 to 20 per cent by weight, preferably about 0.2 to 10 per cent by weight, where, in the ready-to-use colourants (A), the compounds of the formula (I) and the ortho-quinone of the formula (II) and/or para-quinone of the formula (III) are in each case present in a total amount of from about 0.01 to 10 per cent by weight, preferably about 0.1 to 5 per cent by weight.

The preparation form for the components (A1) and (A2) and of the ready-to-use colourant (A) can, for example, be a solution, in particular an aqueous or aqueous-alcoholic solution, a cream, a gel or an emulsion. Its composition represents a mixture of the compound of the formula (I) or of the ortho-quinones of the formula (II) and/or para-quinones of the formula (III) with the additives customary for such preparations.

Customary additives used in colourants in solutions, creams, emulsions, gels or aerosol foams are, for example, solvents, such as water, lower aliphatic alcohols, for example ethanol, n-propanol and isopropanol or glycols, such as glycerol and 1,2-propanediol, and also wetting agents or emulsifiers from the classes of anionic, cationic, amphoteric or nonionogenic surface-active substances, such as fatty alcohol sulphates, oxethylated fatty alcohol sulphates, alkylsulphonates, alkylbenzene-sulphonates, alkyltrimethylammonium salts, alkylbetaines, oxethylated fatty alcohols, oxethylated nonylphenols, fatty acid alkanolamides, oxethylated fatty acid esters, and also thickeners, such as higher fatty alcohols, starch or cellulose derivatives, perfumes, hair pretreatment agents, conditioners, hair-swelling agents, preservatives, and also Vaseline, paraffin oil and fatty acids, and also care substances, such as cationic resins, lanolin derivatives, cholesterol, pantothenic acid and betaine. The constituents mentioned are used in the amounts customary for such purposes, for example the wetting agents and emulsifiers in concentrations of from about 0.5 to 30 per cent by weight (in each case based on component (A1) or (A2)), the thickeners in an amount of from about 0.1 to 25 per cent by weight (each case based on component (A1) or (A2) or (A")) and the care substances in a concentration of from about 0.1 to 5.0 per cent by weight (in each case based on component (A1) or (A2) or (A")).

The pH of the ready-to-use colourant (A) and of the colour carrier masses (A1), (A2) and (A") is in each case about 3 to 12, preferably about 4 to 10, the pH of the ready-to-use colourant (A) generally being established during the mixing of the individual components. To establish the pH of components (A1), (A2) and (A") and of the ready-to-use colourant (A) desired for the colouring it is, however, also possible, if necessary, to add alkaline agents, such as, for example, alkali metal hydroxides, alkaline earth metal hydroxides, alkali metal acetates (in particular sodium acetate), alkaline earth metal acetates, alkali metal carbonates or alkaline earth metal carbonates (in particular sodium carbonate), or acids, such as, for example, lactic acid, acetic acid, tartaric acid, phosphoric acid, hydrochloric acid, citric acid, ascorbic acid or boric acid.

The ready-to-use colourant is prepared directly prior to application by mixing the components (A1) and (A2) or the component (A') with water or the component (A") - if necessary with the addition of an alkalinizing agent or acid - and then applied to the fibres, in particular human hair. Depending on the desired depth of colour, this mixture is left to act for about 5 to 60 minutes, preferably about 15 to 30 minutes, at a temperature of from about 20 to 50°C, in particular at about 30 to 40°C. The fibre is then rinsed with water, optionally washed with a shampoo and then dried.

The present invention further provides a multicomponent kit consisting of an agent of component (A1) and an agent of component (A2), and optionally an agent for adjusting the pH (alkalinizing agent or acid). The agents of components (A1) and (A2) can of course also consist of two or more individual components which are mixed together directly prior to application. A 2-component kit is likewise possible, the 1 st component of which consists of a powder (A') comprising the compounds of the formula (I) and the ortho-quinones of the formula (II) and/or para-quinones of the formula (III) and optionally further customary pulverulent cosmetic additives, and whose 2nd component is water or a liquid cosmetic preparation (A"). Particularly preferred is a 2-component kit whose 1 st component consists of a powder (A') comprising the compounds of the formula (I) and the ortho-quinones of the formula (II) and/or para-quinones of the formula (III), and optionally further customary pulverulent cosmetic additives, and whose 2nd component is water or a liquid cosmetic preparation (A").

The colourant according to the invention permits a gentle, even and long-lasting colouration of the fibres, in particular of keratin fibres, such as, for example, human hair, with a broad colour palette from yellow to blue colour shades being possible.

The examples below are intended to illustrate the subject-matter in more detail without limiting it to these examples.

### Examples

### Examples 1 to 13: Hair colourants

| Component (A1) | |
|---|---|
| 4.0 g | Decylpolyglucose (Plantaren® 2000), aqueous solution |
| 0.2 g | Ethylenediaminotetraacetic acid disodium salt hydrate |
| 5.0 g | Ethanol |
| 0.45 g | 3,4-Dimethyl-2(3H)-thiazolone hydrazone hydrochloride |
| ad 100.0 g | water, demineralized |

| Component (A2) | |
|---|---|
| X g | ortho- or para-quinone as in Table 1 |

At room temperature (20-30 °C) or with gentle heating (35-45 °C), the abovementioned constituents are mixed together homogeneously. The pH of the ready-to-use colourant (A) is - if necessary - adjusted to the value given in Table 1 using sodium hydroxide solution, sodium carbonate, ammonia or citric acid.

The ready-to-use hair colourant is applied to the hair and distributed evenly using a brush. After a contact time of 30 minutes at 40 °C, the hair is rinsed with lukewarm water and then dried.

The amount of ortho- or para-quinones used and the resulting colourations are summarized in Table 1 below.

**Table 1:**

| **Ex. No.** | **Quinone used** | **pH** | **Colour shade** | **Colour measurement values** |
|---|---|---|---|---|
| **1** | 4,5-Dimethoxy-3,5-cyclohexadiene-1,2-dione (0.41 g) | 6.0 | intense red | L= 34.7 |
| | | | | a= +27.8 |
| | | | | b= +18.0 |
| **2** | 5(5H),6(6H)-Dioxo-1,3-benzodioxol (0.39 g) | 6.2 | intense garnet | L= 23.0 |
| | | | | a= +18.4 |
| | | | | b= + 5.0 |
| **3** | Cacotheline (1.26 g) | 8.9 | violet | L= 32.1 |
| | | | | a= +22.6 |
| | | | | b= - 4.1 |
| **4** | 1,2-Naphthalene-dione (0.38 g) | 6.1 | intense aubergine | L= 27.7 |
| | | | | a= +27.8 |
| | | | | b= -11.8 |
| **5** | 1,2-Naphthalenedione-4-sulphonic acid sodium salt (0.66 g) | 3.5 | violet | L= 35.1. |
| | | | | a= +26.4 |
| | | | | b= -19.7 |
| **6** | 4-Amino-1,2-naphthalenedione hydrate(2:1) (0.47 g) | 6.5 | intense red | L= 43.8 |
| | | | | a= +31.9 |
| | | | | b= +25.4 |
| **7** | 9,10-Phenanthrene quinone (0.55 g) | 6.3 | pale orange | L= 53.3 |
| | | | | a= +17.8 |
| | | | | b= +27.1 |
| **8** | 1,4-Benzoquiinone (0.27 g) | 5.7 | intense violet- black | L= 27.9 |
| | | | | a=+ 3.4 |
| | | | | b= - 0.6 |
| **9** | 2-Methyl-1,4-benzoquinone (0.30 g) | 6.1 | intense brown | L= 26.9 |
| | | | | a= + 2.8 |
| | | | | b= + 2.5 |
| **10** | 2,5-Dihydroxy-1,4-benzoquinone (0.35 g) | 3.0 | intense ruby red | L= 35.1 |
| | | | | a= +43.4 |
| | | | | b= +23.4 |
| **11** | 2,5-Di((2-hydroxyethyl)amino)-1,4-benzoquinone (0.56 g) | 9.1 | yellow | L= 68.2 |
| | | | | a= + 2.9 |
| | | | | b= +38.3 |
| **12** | 1,4-Naphthalenedione (0.39 g) | 6.8 | intense brown | L= 39.9 |
| | | | | a= +11.2 |
| | | | | b= +14.1 |
| **13** | 5,8-Dihydroxy-1,4-naphthalenedione (0.48 g) | 9.1 | intense brown | L= 36.1 |
| | | | | a= +10.9 |
| | | | | b= +11.0 |

### Examples 14 to 22: Hair colourants

| Component (A1) | |
|---|---|
| 4.0 g | Decylpolyglucose (Plantaren® 2000), aqueous solution |
| 0.2 g | Ethylenediaminotetraacetic acid disodium salt hydrate |
| 5.0 g | Ethanol |
| 0.48 g | 3,4,5-Trimethyl-2(3H)-thiazolone hydrazone hydrochloride |
| ad 100.0 g | Water, demineralized |

| Component (A2) | |
|---|---|
| X g | ortho- or para-quinone as in Table 2 |

At room temperature (20-30 °C) or with gentle heating (35-45 °C), the abovementioned constituents are mixed together homogeneously. The pH of the ready-to-use colourant (A) is - if necessary - adjusted to the value given in Table 2 using sodium hydroxide solution, sodium carbonate, ammonia or citric acid.

The ready-to-use hair colourant is applied to the hair and distributed evenly using a brush. After a contact time of 30 minutes at 40 °C, the hair is rinsed with lukewarm water and then dried.

The amount of ortho- or para-quinones used and the resulting colourations are summarized in Table 2 below.

**Table 2:**

| **Ex. No.** | **Quinone used** | **pH** | **Colour shade** | **Colour measurement values** |
|---|---|---|---|---|
| **14** | 4,5-Dimethoxy-3,5-cyclohexadiene- 1,2-dione (0.41 g) | 6.1 | intense garnet red | L= 30.5 |
| | | | | a= +20.7 |
| | | | | b= + 9.2 |
| **15** | 5(5H),6(6H)-Dioxo-1,3-benzodioxol (0.39 g) | 6.6 | violet-grey | L= 30.5 |
| | | | | a= + 5.3 |
| | | | | b= - 2.0 |
| **16** | Cacotheline (1.26 g) | 9.0 | blue | L= 33.4 |
| | | | | a= +16.3 |
| | | | | b= -12.1 |
| **17** | 1,2-Naphthalenedione (0.38 g) | 6.2 | intense aubergine | L= 29.5 |
| | | | | a= +18.1 |
| | | | | b= - 5.6 |
| **18** | 4-Amino-1,2-naphthalenedione hydrate (2:1) (0.47 g) | 6.2 | intense red-garnet | L= 44.1 |
| | | | | a= +20.4 |
| | | | | b= +17.9 |
| **19** | 9,10-Phenanthrenequinone (0.55 g) | 6.4 | pale orange | L= 58.0 |
| | | | | a= + 5.8 |
| | | | | b= +13.7 |
| **20** | 2-Methyl-1,4-benzoquinone (0.30 g) | 5.4 | pale brown | L= 52.4 |
| | | | | a= - 1.2 |
| | | | | b= +16.2 |
| **21** | 2,5-Dihydroxy-1,4-benzoquinone (0.35 g) | 3.2 | intense garnet red | L= 38.6 |
| | | | | a= +40.3 |
| | | | | b= +20.2 |
| **22** | 1H-Indol-4,7-dione (0.37 g) | 6.0 | intense garnet | L= 28.9 |
| | | | | a= +16.5 |
| | | | | b= + 9.9 |

The L*a*b* colour measurement values given in the present examples were determined using a colour measuring instrument from Minolta, model Chromameter II. Here, the L value is the lightness (i.e. the lower the L value, the greater the colour intensity), while the a value is a measure of the red fraction (i.e. the greater the a value, the greater the red fraction). The b value is a measure of the blue fraction of the colour, the greater the blue fraction, the more negative the b value.

## Claims

1. Agent for colouring keratin fibres (A) which is prepared by mixing two components (A1) and (A2) and is **characterized in that** component (A1) comprises at least one heterocyclic hydrazone derivative of the formula (I) or its physiologically compatible salt, 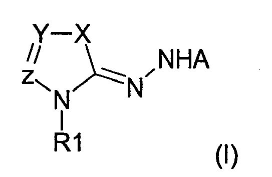 in which
**X** is oxygen, sulphur or N-R2,
**Y** is C-R3 or nitrogen and
**Z** is C-R4 or nitrogen,
with the proviso that the heterocyclic moiety in the formula (I) has at most three heteroatoms;
**A** is hydrogen, an acetyl group, a trifluoroacetyl group, a formyl group, a (C₁-C₆)-alkylsulphonyl group or an arylsulphonyl group;
**R1** and **R2** may be identical or different, and, independently of one another, are a saturated or unsaturated (C₁-C₁₂)-alkyl group, a (C₁-C₁₂)-alkyl group substituted by a halogen atom, a hydroxy-(C₂-C₁₂)-alkyl group, an amino-(C₂-C₁₂)-alkyl group, a sulphonic acid-(C₁-C₁₂)-alkyl group, an isocyclic or heterocyclic group, a formyl group, a C(O)-alkyl group, a C(O)-phenyl group, a C(O)NH-alkyl group, a C(O)NH-phenyl group, a substituted or unsubstituted phenyl group or a substituted or unsubstituted benzyl group;
**R3** and **R4** may be identical or different and, independently of one another, are hydrogen, a halogen atom, a saturated or unsaturated (C₁₋C₁₂)-alkyl group, a (C₁-C₁₂)-alkyl group substituted by a halogen atom, a hydroxyl group, a hydroxy-(C₁-C₁₂)-alkyl group, a (C₁-C₁₂)-alkoxy group, a (C₁-C₆)-alkoxy-(C₁-C₆)-alkyl group, a cyano group, a nitro group, an amino group, a (C₁-C₁₂)-alkylamino group, a (C₁-C₁₂)-dialkylamino group, an isocyclic or heterocyclic group, a carboxylic acid, a C(O)O-(C₁-C₁₂)-alkyl group, a substituted or unsubstituted C(O)O-phenyl group, a substituted or unsubstituted phenyl group or a naphthyl group;
and if **Y** and **Z** are C-R3 and C-R4, **R3** and **R4,** together with the residual molecule, can form a heterocyclic, saturated or unsaturated, substituted or unsubstituted ring system;
and the component (A2) comprises at least one ortho-quinone of the formula (II) or para-quinone of the formula (III), 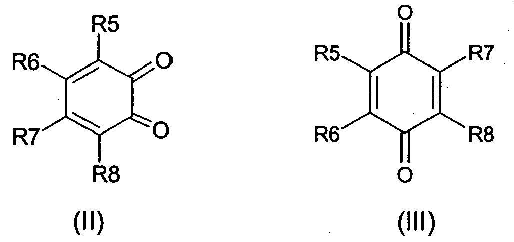
where **R5, R6, R7** and **R8,** independently of one another, are hydrogen, a halogen atom, a (C₁-C₁₂)-alkyl group substituted by a halogen atom, a hydroxyl group, a hydroxy-(C₁-C₁₂)-alkyl group, a polyhydroxy-(C₂-C₁₂)-alkyl group, a (C₁-C₁₂)-alkoxy group, a mono-(C₁-C₆)-alkoxy-(C₁-C₆)-alkyl group, a poly-(C₁-C₆)-alkoxy-(C₂-C₆)-alkyl group, a (C₁- C₂)-alkylenedioxy group, a cyano group, a nitro group, a sulphonic acid group, a carboxylic acid, a C(O)O-(C₁-C₁₂)-alkyl group, a substituted or unsubstituted C(O)O-phenyl group, a substituted or unsubstituted phenyl group, a naphthyl group, an isocyclic or heterocyclic compound or an amino group **-NR9R10,** where the radicals **R9** and **R10** may be identical or different and, independently of one another, are hydrogen, a (C₁-C₁₂)-alkyl group, a carbocyclic or heterocyclic substituted aromatic compound or a carbocyclic or heterocyclic unsubstituted aromatic compound, or **R9** and **R10,** together with the nitrogen atom, form a saturated or unsaturated, substituted or unsubstituted heterocyclic (C₃-C₆)-radical;
or in the case of o-quinones of the formula (II) **R5** and **R6** and/or **R6** and **R7** and/or **R7** and **R8,** or in the case of p-quinones of the formula (III) **R5** and **R6,** and/or **R7** and **R8,** in each case together with the remaining molecule, can form a heterocyclic or carbocyclic, substituted or unsubstituted ring.

2. Agent for colouring keratin fibres (A) which is prepared by mixing two components (A') and (A") and is **characterized in that** component (A') is present in solid form and comprises at least one heterocyclic hydrazone derivative of the formula (I) or its physiologically compatible salt, 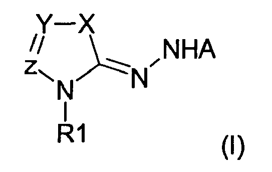 in which **X** is oxygen, sulphur or N-R2,
**Y** is C-R3 or nitrogen and
**Z** is C-R4 or nitrogen,
with the proviso that the heterocyclic moiety in the formula (I) has at most three heteroatoms;
**A** is hydrogen, an acetyl group, a trifluoroacetyl group, a formyl group, a (C₁-C₆)-alkylsulphonyl group or an arylsulphonyl group;
**R1** and **R2** may be identical or different, and, independently of one another, are a saturated or unsaturated (C₁-C₁₂)-alkyl group, a (C₁-C₁₂)-alkyl group substituted by a halogen atom, a hydroxy-(C₂-C₁₂)-alkyl group, an amino-(C₂-C₁₂)-alkyl group, a sulphonic acid-(C₁-C₁₂)-alkyl group, an isocyclic or heterocyclic group, a formyl group, a C(O)-alkyl group, a C(O)-phenyl group, a C(O)NH-alkyl group, a C(O)NH-phenyl group, a substituted or unsubstituted phenyl group or a substituted or unsubstituted benzyl group;
**R3** and **R4** may be identical or different and, independently of one another, are hydrogen, a halogen atom, a saturated or unsaturated (C₁₋C₁₂)-alkyl group, a (C₁-C₁₂)-alkyl group substituted by a halogen atom, a hydroxyl group, a hydroxy-(C₁-C₁₂)-alkyl group, a (C₁-C₁₂)-alkoxy group, a (C₁-C₆)-alkoxy-(C₁-C₆)-alkyl group, a cyano group, a nitro group, an amino group, a (C₁-C₁₂)-atky)amino group, a (C₁-C₁₂)-dialkylamino group, an isocyclic or heterocyclic group, a carboxylic acid, a C(O)O-(C₁-C₁₂)-a)kyl group, a substituted or unsubstituted C(O)O-phenyl group, a substituted or unsubstituted phenyl group or a naphthyl group;
and if **Y** and **Z** are C-R3 and C-R4, **R3** and **R4,** together with the residual molecule, can form a heterocyclic, saturated or unsaturated, substituted or unsubstituted ring system;
and the component (A2) comprises at least one ortho-quinone of the formula (II) or para-quinone of the formula (III), 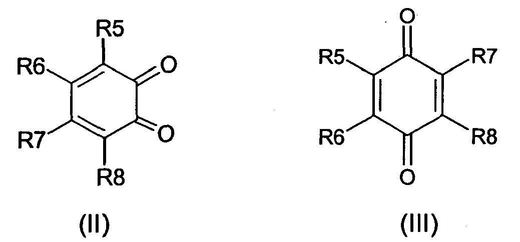
where **R5, R6, R7** and **R8,** independently of one another, are hydrogen, a halogen atom, a (C₁-C₁₂)-alkyl group substituted by a halogen atom, a hydroxyl group, a hydroxy-(C₁-C₁₂)-alkyl group, a polyhydroxy-(C₂-C₁₂)-alkyl group, a (C₁-C₁₂)-alkoxy group, a mono-(C₁-C₆)-alkoxy-(C₁-C₆)-alkyl group, a poly-(C₁-C₆)-alkoxy-(C₂-C₆)-alkyl group, a (C₁- C₂)-alkylenedioxy group, a cyano group, a nitro group, a sulphonic acid group, a carboxylic acid, a C(O)O-(C₁-C₁₂)-alkyl group, a substituted or unsubstituted C(O)O-phenyl group, a substituted or unsubstituted phenyl group, a naphthyl group, an isocyclic or heterocyclic compound or an amino group **-NR9R10,** where the radicals **R9** and **R10** may be identical or different and, independently of one another, are hydrogen, a (C₁-C₁₂-alkyl group, a carbocyclic or heterocyclic substituted aromatic compound or a carbocyclic or heterocyclic unsubstituted aromatic compound, or **R9** and **R10,** together with the nitrogen atom, form a saturated or unsaturated, substituted or unsubstituted heterocyclic (C₃-C₆)-radical;
or in the case of o-quinones of the formula (II) **R5** and **R6** and/or **R6** and **R7** and/or **R7** and **R8,** or in the case of p-quinones of the formula (III) **R5** and **R6,** and/or **R7** and **R8,** in each case together with the remaining molecule, can form a heterocyclic or carbocyclic, substituted or unsubstituted ring;
and component (A") is water or a liquid preparation comprising the other constituents of the agent.

3. Agent according to Claim 1 or 2, **characterized in that**, in the hydrazone derivatives of the formula (I), **X** is sulphur, **Y** is C-R3, **Z** is C-R4 and A is hydrogen.

4. Agent according to one of Claims 1 to 3, **characterized in that** the hydrazone derivative of the formula (I) is chosen from 3-methyl-2(3H)-thiazolone hydrazone, 3,4-dimethyl-2(3H)-thiazolone hydrazone, 4-tert-butyl-3-methyl-2(3H)-thiazolone hydrazone, 3-methyl-4-phenyl-2(3H)-thiazolone hydrazone, 3-methyl-4-(4-tolyl)-2(3H)-thiazolone hydrazone, 4-(4-methoxy)phenyl-3-methyl-2(3H)-thiazolone hydrazone, 4-(4-ethoxy)phenyl-3-methyl-2(3H)-thiazolone hydrazone, 4-(4-bromophenyl)-3-methyl-2(3H)-thiazolone hydrazone, 4-(3-bromophenyl)-3-methyl-2(3H)-thiazolone hydrazone, 4-(4-chlorophenyl)-3-methyl-2(3H)-thiazolone hydrazone, 4-(3-chlorophenyl)-3-methyl-2(3H)-thiazolone hydrazone, 3-methyl-4-(4-nitrophenyl)-2(3H)-thiazolone hydrazone, 3-methyl-4-(3-nitrophenyl)-2(3H)-thiazolone hydrazone, 4-([1,1`-biphenyl]-4-yl)-3-methyl-2(3H)-thiazolone hydrazone, ethyl 2-hydrazono-2,3-dihydro-3-methyl-4-thiazolecarboxylate, 3,4,5-trimethyl-2(3H)-thiazolone hydrazone, 3,4-dimethyl-5-phenyl-2(3H)-thiazolone hydrazone, 3,5-dimethyl-4-phenyl-2(3H)-thiazolone hydrazone, 3-methyl-4,5-diphenyl-2(3H)-thiazolone hydrazone, 5-ethyl-3-methyl-4-phenyl-2(3H)-thiazolone hydrazone, 4-(4-bromophenyl)-3-methyl-5-phenyl-2(3H)-thiazolone hydrazone, 3-methyl-5-phenyl-4-(4-tolyl)-2(3H)-thiazolone hydrazone, 5-(4-chlorophenyl)-4-phenyl-3-methyl-2(3H)-thiazolone hydrazone, 5-(4-chlorophenyl)-4-(4-methoxyphenyl)-3-methyl-2(3H)-thiazolone hydrazone, ethyl 2-hydrazono-2,3-dihydro-3,4-dimethyl-4-thiazolecarboxylate, 4-amino-2-hydrazono-2,3-dihydro-3-methyl-5-thiazolecarbonitrile, 3-ethyl-4,5-dimethyl-2(3H)-thiazolone hydrazone, ethyl 2-hydrazono-2,3-dihydro-3-ethyl-4-methyl-thiazolecarboxylate, 5-methyl-3-(1-methylethyl)-4-phenyl-2(3H)-thiazolone hydrazone, 4,5-dimethyl-3-(1-methylethyl)-2(3H)-thiazolone hydrazone, 3-(1-methylethyl)-4,5-diphenyl-2(3H)-thiazolone hydrazone, 4,5-dimethyl-3-propyl-2(3H)-thiazolone hydrazone, 4,5-diphenyl-3-propyl-2(3H)-thiazolone hydrazone, 3-butyl-4,5-dimethyl-2(3H)-thiazolone hydrazone, 3-butyl-4,5-diphenyl-2(3H)-thiazolone hydrazone, 4,5-dimethyl-3-(2-methylpropyl)-2(3H)-thiazolone hydrazone, 3-(2-methylpropyl)-4,5-diphenyl-2(3H)-thiazolone hydrazone, 3-hydroxyethyl-2(3H)-thiazolone hydrazone, 3-hydroxyethyl-4-methyl-2(3H)-thiazolone hydrazone, 3-hydroxyethyl-4,5-dimethyl-2(3H)-thiazolone hydrazone, 3-aminoethyl-2(3H)-thiazolone hydrazone, 3-aminoethyl-4-methyl-2(3H)-thiazolone hydrazone, 3-aminoethyl-4,5-dimethyl-2(3H)-thiazolone hydrazone, 3,4-diphenyl-2(3H)-thiazolone hydrazone, 4-methyl-3-phenyl-2(3H)-thiazolone hydrazone, 4,5-dimethyl-3-phenyl-2(3H)-thiazolone hydrazone, 4-p-biphenylyl-3-phenyl-2(3H)-thiazolone hydrazone, 4-(4-methoxy)phenyl-3-phenyl-2(3H)-thiazolone hydrazone, 4-tert-butyl-3-phenyl-2(3H)-thiazolone hydrazone, 5-methyl-3,4-diphenyl-2(3H)-thiazolone hydrazone, 3,4,5-triphenyl-2(3H)-thiazolone hydrazone, 4,5-dimethyl-3-(phenylmethyl)-2(3H)-thiazolone hydrazone, 3-(2-propenyl)-2(3H)-thiazolone hydrazone, 4-methyl-3-(2-propenyl)-2(3H)-thiazolone hydrazone, 4-tert-butyl-3-(2-propenyl)-2(3H)-thiazolone hydrazone, 4-phenyl-3-(2-propenyl)-2(3H)-thiazolone hydrazone, 4,5-dimethyl-3-(2-propenyl)-2(3H)-thiazolone hydrazone, 4,5-diphenyl-3-(2-propenyl)-2(3H)-thiazolone hydrazone and ethyl 2-hydrazono-2,3-dihydro-3-[(phenylamino)carbonyl]-4-methylthiazolecarboxylate.

5. Agent according to one of Claims 1 to 4, **characterized in that** the ortho-quinone of the formula (II) is chosen from 4,5-dimethoxy-3,5-cyclohexadiene-1,2-dione, 5(5H),6(6H)-dioxo-1,3-benzodioxol, 2,3-dihydro-1,4-dimethy)-3-hydroxy-1H-indole-5,6-dione, 2,3-dihydro-3-hydroxy-4-methoxy-1-methyl-1H-indole-5,6-dione, 3,5-di(1,1-dimethylethyl)-3,5-cyclohexadiene-1,2-dione, 2,3-dihydro-3-hydroxy-1-methyl-1H-indole-5,6-dione, 3,4,5,6-tetrachloro-3,5-cyclohexadiene-1,2-dione, 4,5-dimethoxy-3,6-diphenyl-3,5-cyclohexadiene-1,2-dione, 4,5-di(phenylamino)-3,5-cyclohexadiene-1,2-dione, cacotheline, 3,4-dihydro-3,4-dioxo-1-naphthalenesulphonic acid ammonium salt, 4-methoxy-1,2-naphthalenedione, 1,2-naphthalenedione, 1,2-naphthalenedione-4-sulphonic acid sodium salt, 4-amino-1,2-naphthalenedione hydrate (2:1) and 9,10-phenanthrenequinone.

6. Agent according to one of Claims 1 to 4, **characterized in that** the para-quinone of the formula (III) is chosen from 1,4-benzoquinone, 2-methyl-1,4-benzoquinone, 2-tert-butyl-1,4-benzoquinone, 2-phenyl-1,4-benzoquinone, 2,5-dimethyl-1,4-benzoquinone, 2,6-dimethyl-1,4-benzoquinone, 2-methyl-5-(1-methylethyl)-1,4-benzoquinone, 2,5-di-tert-butyl-1,4-benzoquinone, 2,6-di-tert-butyl-1,4-benzoquinone, 2,5-diphenyl-1,4-benzoquinone, 2,3,5,6-tetramethyl-1,4-benzoquinone, 2-methoxy-1,4-benzoquinone, 2-methoxy-5-methyl-1,4-benzoquinone, 2,5-dimethoxy-1,4-benzoquinone, 2,6-d imethoxy- 1,4-benzoquinone, 2,3-dimethoxy-5-methyl-1,4-benzoquinone, 2,6-dimethoxy-5-methyl-1,4-benzoquinone, 2,5-dihydroxy-1,4-benzoquinone, 2,5-dihydroxy-3-methyl-1,4-benzoquinone, 2,5-dihydroxy-3-methoxy-6-methyl-1,4-benzoquinone, 2,5-dihydroxy-3,6-diphenyl-1,4-benzoquinone, 3-hydroxy-2-methoxy-5-methyl-1,4-benzoquinone, 2-hydroxymethyl-6-methoxy-1,4-benzoquinone, 2,3,5,6-tetrahydroxy-1,4-benzoquinone, 2-bromo-1,4-benzoquinone, 2-chloro-1,4-benzoquinone, 2-fluoro-1,4-benzoquinone, 2-bromo-5-methyl-1,4-benzoquinone, 2,5-dibromo-1,4-benzoquinone, 2,5-dibromo-3,6-diphenyl-1,4-benzoquinone, 2,5-dibromo-3-methyl-6-(1-methylethyl)-1,4-benzoquinone, 2-chloro-6-methyl-1,4-benzoquinone, 2,5-dichloro-1,4-benzoquinone, 2,6-dichloro-1,4-benzoquinone, 2,5-dichloro-3,6-dimethyl-1,4-benzoquinone, 2,3-dichloro-5,6-dicyano-1,4-benzoquinone, 3,6-dioxocyclohexa-1,4-diene-1,2-dicarbonitrile, 2,5-dichloro-3,6-dioxocyclohexa-1,4-diene-1,4-dicarbonitrile, 2,5-dichloro-3,6-dihydroxy-1,4-benzoquinone, 2,5-dichloro-3,6-dimethylamino-1,4-benzoquinone, 2,3,5,6-tetrabromo-1,4-benzoquinone, 2,3,5,6-tetrachloro-1,4-benzoquinone, 2,3,5,6-tetrafluoro-1,4-benzoquinone, 2-amino-5-methyl-1,4-benzoquinone, 5-amino-2-chloro-1,4-benzoquinone, 2,5-di((2-hydroxyethyl)amino)-1,4-benzoquinone, 2,5-diamino-3,6-dichloro-1,4-benzoquinone, 1,4-naphthalenedione, 5,8-dihydroxy-1,4-naphthalenedione and 1 H-indole-4,7-dione.

7. Agent according to one of Claims 1 to 6, **characterized in that** it comprises the heterocyclic hydrazone derivatives of the formula (I) and the ortho-quinones of the formula (II) and/or para-quinones of the formula (III) in the particular colour carrier mass (component (A1) or component (A2) or component (A')) in each case in a total amount of from 0.02 to 20 per cent by weight.

8. Agent according to one of Claims 1 to 7, **characterized in that** it additionally comprises 0.02 to 20 per cent by weight of a physiologically acceptable direct dye from the group of cationic and anionic dyes, dispersion dyes, nitro dyes, azo dyes, quinone dyes and triphenylmethane dyes.

9. Agent according to one of Claims 1 to 8, **characterized in that** the ready-to-use colourant (A) has a pH of from 3 to 12.

10. Agent according to one of Claims 1 to 9, **characterized in that** it is a hair colourant.

11. Method of colouring hair in which the ready-to-use colourant (A) is prepared directly prior to application by mixing two components (A1) and (A2) or (A') and (A"), if necessary with the addition of an alkalinizing agent or an acid, and is then applied to the hair and, after a contact time of from 5 to 60 minutes at a temperature of from 20 to 50 °C, the hair is rinsed with water, if necessary washed with a shampoo and then dried, **characterized in that** a colourant according to one of Claims 1 to 10 is used.

12. Multicomponent kit consisting of an agent of component (A1) and an agent of component (A2), and if necessary an agent for adjusting the pH, **characterized in that** the component (A1) comprises at least one heterocyclic hydrazone derivative of the formula (I) or its physiologically compatible salt according to one of Claims 1 to 4 and the component (A2) comprises at least one ortho-quinone of the formula (II) or para-quinone of the formula (III) according to one of Claims 1, 5 and 6.

13. Multicomponent kit consisting of an agent of component (A') and an agent of component (A"), **characterized in that** the component (A') is a powder comprising at least one compound of the formula (I) and at least one ortho-quinone compound of the formula (II) and/or para-quinone compound of the formula (III), and optionally further customary pulverulent cosmetic additives, and the component (A") is water or a liquid cosmetic preparation.
